(19) Europäisches Patentamt
European Patent Office
Office européen des brevets

(11) Publication number: **0 119 450 A1**

# (12) EUROPEAN PATENT APPLICATION

(21) Application number: **84101395.6**

(22) Date of filing: **10.02.84**

(51) Int. Cl.³: **C 07 D 231/06**, C 07 D 401/04, A 61 K 31/415

(30) Priority: **11.02.83 GB 8303787**
**11.02.83 GB 8303788**

(43) Date of publication of application: **26.09.84**
**Bulletin 84/39**

(84) Designated Contracting States: **AT BE CH DE FR GB IT LI LU NL SE**

(71) Applicant: **THE WELLCOME FOUNDATION LIMITED, 183-193 Euston Road, London NW1 2BP (GB)**

(72) Inventor: **Islip, Peter John, 35 Arkwright Road, Sanderstead Surrey (GB)**
Inventor: **Copp, Frederick Charles, "Rotherwood" 32 Stanley Avenue, Beckenham Kent (GB)**
Inventor: **Kneen, Geoffrey, 119 Lennard Road, Beckenham Kent (GB)**

(74) Representative: **Sandmair, Kurt, Dr. et al, Dr. Berg, Dipl.-Ing. Stapf Dipl.-Ing. Schwabe, Dr. Dr. Sandmair Mauerkircherstrasse 45, D-8000 München 80 (DE)**

## (54) Preparation of aminopyrazolines and intermediates used therein.

(57) A process for the preparation of 3-(N-substituted amino)-2-pyrazolines, of formula (I)

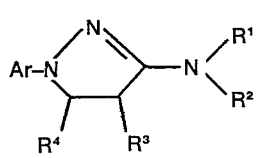

(I)

which comprises
(i) reacting of a compound of formula (II)

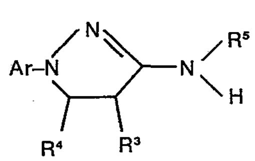

(II)

with a compound serving to effect substitution of a group R¹ on the amino group attached to the 3-position of the pyrazoline ring in the compound of formula (II), thereby to form a compound of formula (III)

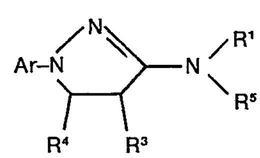

(III)

and converting the compound of formula (III) to a compound of formula (I) or an acid addition salt thereof.

Certain of the intermediates of formula (III) are novel and possess pharmacological activity.

EP 0 119 450 A1

## CHEMICAL PROCESS AND INTERMEDIATES FOR USE THEREIN

The present invention relates to a process for the preparation of substituted amino-pyrazolines, to certain intermediate compounds in the process which are novel, pharmaceutical formulations containing such compounds and their use in medicine.

Certain 3-substituted amino-2-pyrazolines are known in the art to exhibit anti-inflammatory activity. Although there are many methods for the preparation of 3-amino-2-pyrazolines, fewer methods are known for the preparation of 3-amino-2-pyrazolines in which the 3-amino group is substituted by one or two substituents.

Duffin and Kendall (J.Chem.Soc. 1954,408) have prepared 1-phenyl-3-ethylamino-2-pyrazoline by treatment of the corresponding 1-phenyl-3-amino-2-pyrazoline with acetic acid in acetic anhydride followed by reduction of the intermediate 1-phenyl-3-acetamido-2-pyrazoline with lithium aluminium hydride. However, the acylation step is low yielding, resulting in an overall low yield of the 3-ethylamino-2-pyrazoline from the unsubstituted 3-amino-2-pyrazoline starting material. Moreover, the use of lithium aluminium hydride on a commercial scale is both hazardous and costly, and requires special precautions and techniques, such as use of anhydrous solvents.

European Patent Specification No. 0 055 418 discloses the preparation of 1-substituted phenyl-3-alkylamino-2-pyrazolines by reduction of the corresponding 3-acylamino-2-pyrazolines. This process suffers the disadvantage of requiring use of lithium aluminium hydride.

Elguero et al (Bull.Soc.Chim.France. 1970, p.1571, 1576) have prepared 3-N-ethylamino-2-pyrazolines by the method of Duffin & Kendall, and attempted to substitute further the amino group by carrying out a second acylation-reduction sequence to form 1-phenyl-3-N,N-diethylamino-2-pyrazoline via the intermediate 1-phenyl-3-N-acetyl-3-N-ethylamino-2-pyrazoline. However, the identity of the product of the acylation reaction was not confirmed, and was thought to be either the desired 1-phenyl-3-N-acetyl-3-N-ethylamino-2-pyrazoline, or, the product formed by acylation of the ring nitrogen instead of the exocylic amino nitrogen; and, the reduction of the intermediate resulted in low yields and mixtures of products, the two major products being identified as 1-phenyl-3-ethylamino-2-pyrazoline and 1-phenyl-2-pyrazoline.

We have now discovered a new process for the preparation of 3-N-substituted amino- and 3-N,N-disubstituted amino-2-pyrazolines which provides acceptable yields, does not result in mixtures of products, and does not require the use of lithium aluminium hydride. The new process also proceeds via intermediates which themselves show advantageous stability and possess valuable pharmacological properties.

In one aspect the present invention provides a process for the preparation of 3-(N-substituted amino)-2-pyrazolines, of formula (I)

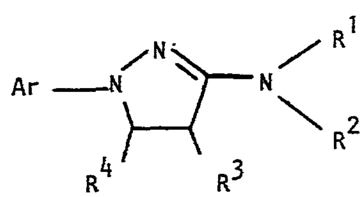

(I)

wherein,

Ar is an aryl group, optionally substituted by one or more suitable substituents;

$R^1$ is alkyl, alkenyl, alkynyl, alkoxy, aryl, aralkyl, aralkenyl or aralkynyl;

$R^2$ is hydrogen, alkyl or aralkyl; and

$R^3$ and $R^4$ are the same or different, each being hydrogen or alkyl,

which comprises

(i) reacting of a compound of formula (II)

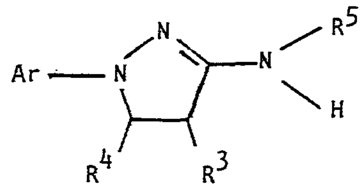

(II)

wherein,

Ar is an aryl group, optionally substituted by one or more suitable substituents;

$R^3$ and $R^4$ are the same or different, and each may be alkyl or hydrogen; and

$R^5$ is acyl or aroyl;

with a compound serving to effect substitution of a group $R^1$ as defined in formula (I) on the amino group attached to the 3-position of the pyrazoline ring in the compound of formula (II), thereby to form a compound of formula (III)

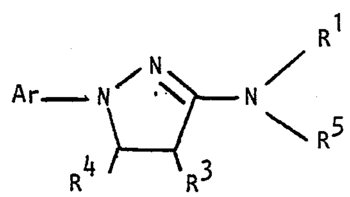

(III)

wherein Ar, $R^1$, $R^3$, $R^4$ and $R^5$ are as hereinbefore defined;

and converting the compound of formula (III) to a compound of formula (I); or an acid addition salt thereof.

Within the definition of compounds of formulae (II) and (III) are also included, where appropriate, the acid addition salts of such compounds. The intermediate compounds of formula (III) may be produced in situ or (preferably) isolated prior to their conversion to compounds of formula (I).

In formulae (I), (II) and (III), and throughout the specification, the following definitions apply unless the context otherwise requires:-

(a) The term "alkyl", either alone or as part of another group e.g. aralkyl, shall be taken to refer to an alkyl or cycloalkyl group having from 1 to 6 carbon atoms, preferably 1 to 4 carbon atoms, which may optionally be substituted in one or more positions by substituents known to those skilled in the art for example, halogen, carboxy, hydroxy, alkoxycarbonyl, amino, monoalkylamino, dialkylamino, monalkylcarbamoyl, dialkylcarbamoyl cycloalkyl of from 3 to 6 carbon atoms, or amide the nitrogen atom of which may be substituted by one or more alkyl groups.

(b) The term "alkenyl", either alone or as part of another group shall be taken to refer to an alkenyl group having from 2 to 6 carbon atoms, preferably 3 to 5 carbon atoms, which may optionally be substituted in one or more positions by substituents known to those skilled in the art, for example as given above in the definition of "alkyl".

(c) The term "alkynyl" either alone or as part of another group shall be taken to refer to an alkynyl group of from 2 to 6 carbon atoms, preferably 3 to 5 carbon atoms, which may optionally be substituted by substituents known to those skilled in the art, for example as given above in the definition of "alkyl".

(d) The term "acyl" shall be taken to refer to a radical of formula $R^aCO$- wherein $R^a$ is an aliphatic moiety. Examples of acyl are formyl, acetyl and propionyl.

(e) The term "aroyl" shall be taken to refer to a radical of formula $R^b$ CO- wherein $R^b$ is an aryl or aryl-containing moiety such as phenyl or benzyl.

(f) The term "aralkyl" shall be taken to refer to an alkyl group substituted by one or more aryl groups. The terms aralkenyl and aralkynyl shall be construed in like fashion.

(g) The term "aryl group" means any group having aromatic character and in particular an aromatic ring or fused ring system (eg. comprising 2, 3 or more rings) and optionally containing one or more hetero atoms, for example selected from nitrogen, oxygen and sulphur. Examples of aryl groups are, phenyl, pyridyl, quinolyl and naphthyl. Preferably the aromatic ring or ring system is substituted in one position only, but substitution may occur in two or more positions of the ring(s) or, in all available positions of the ring(s). In either case, where more than one substituent is present, each substituent may be the same or different. The term "aryl", either alone or as part of another group e.g. aroyl or aralkyl, shall be construed accordingly.

(h) "Suitable substituents" include those known to persons skilled in the art for the substitution of aromatic ring systems, for example, halo, alkyl,

trihaloalkyl, carboxy, alkoxy, hydroxy, alkylthio, amino (optionally substituted by one or more alkyl groups), alkylsulphinyl and alkylsulphonyl (the alkyl group of which may be substituted by one or more halogen groups).

The process according to the present invention may in particular be used for the preparation of compounds of formula (I) wherein:-

Ar represents a phenyl, pyridyl, quinolyl or naphthyl group any of which may be optionally substituted by one or more groups selected from $C_{1-4}$ alkyl, $C_{3-6}$ alkenyl, $C_{3-6}$ alkynyl, $C_{1-4}$ alkoxy (any of alkyl, alkenyl, alkynyl or alkoxy being optionally substituted by one or more halogen atoms), halo (i.e. fluoro, chloro, bromo or iodo), nitro, amino, hydroxy, carboxy, hydroxy- $C_{1-4}$-alkyl, aryl, $C_{1-4}$ alkylthio, $C_{3-6}$ alkenylthio, arylthio, $C_{1-4}$ alkylsulphinyl, $C_{3-6}$ alkenylsulphinyl, arylsulphinyl, $C_{1-4}$-alkylsulphonyl, $C_{3-6}$ alkenylsulphonyl, and arylsulphonyl;

$R^1$ represents a group selected from $C_{1-4}$ alkyl, $C_{3-6}$ alkenyl, $C_{3-6}$ alkynyl, $C_{1-4}$ alkoxy, phenyl, phenyl-$C_{1-4}$ alkyl, phenyl-$C_{1-4}$ alkenyl, phenyl-$C_{1-4}$ alkynyl, (any of phenyl, phenylalkyl, phenylalkenyl and phenyalkynyl being optionally substituted in the benzene ring by one or more of $C_{1-4}$ alkyl, $C_{3-6}$ alkenyl, $C_{3-6}$ alkynyl, $C_{1-4}$ alkoxy, halo or hydroxy groups), carboxy-$C_{1-4}$ alkyl, $C_{1-4}$ alkoxycarbonyl-$C_{1-4}$ alkyl, cyclo-$C_{3-6}$ alkyl, N-$C_{1-4}$ alkylamino-$C_{1-4}$ alkyl, N,N-di-$C_{1-4}$ alkylamino-$C_{1-4}$ alkyl, N-$C_{1-4}$ alkylcarbamoylalkyl and N, N-di-$C_{1-4}$ alkylcarbamoyl-$C_{1-4}$ alkyl;

$R^2$ represents hydrogen or $C_{1-4}$ alkyl; and

$R^3$ and $R^4$ are the same or different and each represents a hydrogen or a $C_{1-4}$ alkyl group;

and acid addition salts thereof.

Particularly preferred is the use of the process according to the present invention for the preparation of compounds of formula (I) wherein

Ar represents an unsubstituted pyridyl, quinolyl or naphthyl group or a phenyl group substituted by one or more groups selected from $C_{1-4}$ alkyl, trihalo-(eg. trifluoro-) $C_{1-4}$ alkyl, halo, nitro and $C_{1-4}$ alkylthio;

$R^1$ represents a group selected from $C_{1-4}$ alkyl, $C_{3-6}$ alkenyl, $C_{3-6}$ alkynyl, phenyl-$C_{1-4}$ alkyl (eg. benzyl) (the phenylalkyl group being optionally substituted by one or more groups selected from halo, $C_{1-4}$ alkoxy and hydroxy), $C_{1-4}$ alkoxycarbonyl-$C_{1-4}$ alkyl, cyclo-$C_{3-6}$ alkyl, N,N-di-$C_{1-4}$-alkylamino-$C_{1-4}$ alkyl and N,N-di- $C_{1-4}$ alkylcarbamoyl-$C_{1-4}$ alkyl;

$R^2$ represents hydrogen; and

$R^3$ and $R^4$ both represent hydrogen;

and acid addition salts thereof.

The process according to the present invention may include the additional step of modifying the substitution pattern of the aryl group by adding one or more substituents (whether or not the aryl group already bears any substituent), removing any existing substituent, or converting each or any exisiting substituent to another desired substituent. It will be appreciated that such modification may be performed as appropriate, at any convenient stage of the process, that is to say the aryl group in a compound of formula (I), (II) or (III) may be so modified. Such modification may be effected by any of the methods known in the art. Where the modification comprises conversion of one or more substituent to (an) other substituent(s), this may comprise:-

(a) Conversion of a carboxy substituent to an ester thereof or to a carboxylic acid derivative substituent such as hydroxyalkyl (eg hydroxymethyl).

(b) Conversion of a hydroxy substituent to an alkoxy substituent by alkylation, eg. methylation.

(c) Conversion of an alkoxy substituent such as methoxy to a hydroxy substituent, eg. by treatment with boron tribromide.

(d) Conversion of a halo (eg. chloro) substituent to an alkoxy substituent.

(e) Oxidation of an alkylthio or alkylsulphinyl substituent to an alkylsulphinyl or alkylsulphonyl substituent as appropriate.

In stage (i) of the above process according to the invention, the agent serving to effect substitution of a group $R^1$ onto the amino group of the compound of formula (II) is preferably a compound of formula $R^1Z$ wherein $R^1$ is as hereinbefore defined and Z is a displaceable group such as halo (i.e chloro, bromo, or iodo) or an arylsulphonyloxy group. This stage of the reaction is preferably performed in the presence of a base, and is suitably carried out in an aprotic solvent, for example N,N-dimethylformamide, at temperatures ranging from ambient to $100^o$, and for periods of from 15 mins to 24 hours. Suitable bases are, for example, alkali metal amides, alkoxides, or preferably alkali metal hydrides; especially preferred is sodium hydride.

Stage (ii) of the process may proceed by hydrolysis of the group $R^5$ in a compound of formula (III), to yield a compound of formula (I) wherein $R^2$ is hydrogen. The hydrolysis may be effected by, for example, an alkali or alkaline earth metal hydroxide, in a polar solvent, or by use of an acid, for example, a dilute mineral acid, or, in the presence of hydrazine at ambient temperature or above.

Alternatively, stage (ii) of the process may proceed by reduction of the group $R^5$ in a compound of formula (III) to yield a compound of formula (I) wherein $R^2$ is alkyl or aralkyl. The reduction may be effected by methods known to those skilled in the art, for example, by use of a reducing agent appropriate to the substituents.

Compounds of formula (II) may be prepared from compounds of formula (IV), wherein Ar, $R^3$ and $R^4$ are as defined for formula (I), by methods known to those skilled in the art, for example, by reaction of a compound of formula (IV), with an agent serving to introduce the group $R^5$ as a substituent on the amino group attached to the pyrazoline ring:-

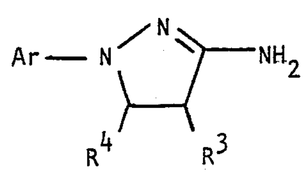

(IV)

Amongst the compounds of formula (III), disclosed hereinbefore as intermediates in the process of the present invention, is a sub-class of compounds of formula (IIIA) (as defined hereinbelow) which are novel and possess valuable pharmacological properties which render them useful in medicine, for example as anti-inflammatory, anti-allergic, analgesic or anti-pyretic agents, or agents for aiding the prevention of tissue rejection.

Thus, the present invention provides a compound of formula (IIIA)

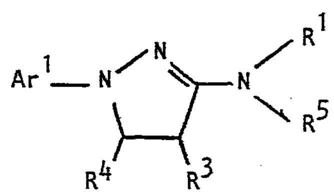

(IIIA)

wherein,

$Ar^1$ is an aryl group optionally substituted by one or more suitable substituents provided that when $Ar^1$ represents phenyl then the phenyl group bears at least one suitable substituent;

$R^1$ is alkyl, alkenyl, alkynyl, aryl, aralkyl, aralkenyl or aralkynyl;

$R^5$ is acyl or aroyl; and

$R^3$ and $R^4$ are the same or different, each being hydrogen or alkyl; and pharmacologically acceptable acid addition salts thereof.

In formula (IIIA) the meanings of "alkyl", "alkenyl", "alkynyl", "acyl", "aroyl", "aralkyl", "aryl group" and "suitable substituent" are as hereinbefore defined.

Preferred compounds of formula (IIIA) include those compounds wherein

$Ar^1$ represents a phenyl, pyridyl, quinolyl or naphthyl group any of which may be optionally substituted by one or more groups selected from $C_{1-4}$ alkyl, $C_{3-6}$ alkenyl, $C_{3-6}$ alkynyl, $C_{1-4}$ alkoxy (any of alkyl, alkenyl, alkynyl or alkoxy being optionally substituted by one or more halogen atoms), halo (i.e. fluoro, chloro, bromo or iodo), nitro, amino, hydroxy, carboxy, hydroxy- $C_{1-4}$-alkyl, aryl, $C_{1-4}$ alkylthio, $C_{3-6}$ alkenylthio, arylthio, $C_{1-4}$ alkylsulphinyl, $C_{3-6}$ alkenylsulphinyl, arylsulphinyl, $C_{1-4}$-alkylsulphonyl, $C_{3-6}$ alkenylsulphonyl and arylsulphonyl;

$R^1$ represents a group selected from $C_{1-4}$ alkyl, $C_{3-6}$ alkenyl, $C_{3-6}$ alkynyl, phenyl, phenyl-$C_{1-4}$ alkyl, phenyl-$C_{3-6}$ alkenyl, phenyl-$C_{3-6}$ alkynyl, (any of phenyl, phenylalkyl, phenylalkenyl and phenyalkynyl being optionally substituted in the benzene ring by one or more of $C_{1-4}$ alkyl, $C_{3-6}$ alkenyl, $C_{3-6}$ alkynyl, $C_{1-4}$ alkoxy, halo, $C_{1-4}$ alkanoyloxy or hydroxy groups), carboxy-$C_{1-4}$ alkyl, $C_{1-4}$ alkoxycarbonyl-$C_{1-4}$ alkyl, cyclo-$C_{3-6}$ alkyl, N-$C_{1-4}$ alkylamino-$C_{1-4}$ alkyl, N,N-di-$C_{1-4}$ alkylamino-$C_{1-4}$ alkyl, N-$C_{1-4}$ alkylcarbamoylalkyl and N, N-di-$C_{1-4}$ alkylcarbamoyl-$C_{1-4}$ alkyl;

$R^5$ represents a group of formula - COQ wherein Q represents hydrogen or a group selected from $C_{1-4}$ alkyl, $C_{3-6}$ alkenyl, $C_{3-6}$ alkynyl (any of alkyl, alkenyl and alkynyl being optionally substituted by one or more halogen atoms), phenyl and phenyl-$C_{1-4}$ alkyl; and

$R^3$ and $R^4$ are the same or different and each represents a hydrogen or a $C_{1-4}$ alkyl group;

and acid addition salts thereof.

Particularly preferred are those compounds of formula (IIIA) wherein

$Ar^1$ represents an unsubstituted pyridyl, quinolyl or naphthyl group or a phenyl group substituted by one or more groups selected from $C_{1-4}$ alkyl, trihalo-(eg. trifluoro-) $C_{1-4}$ alkyl, halo, nitro and $C_{1-4}$ alkylthio;

$R^1$ represents a group selected from $C_{1-4}$ alkyl, $C_{3-6}$ alkenyl, $C_{3-6}$ alkynyl, phenyl $C_{1-4}$ alkyl (eg. benzyl) (the phenylalkyl group being optionally substituted by one or more groups selected from halo, $C_{1-4}$ alkoxy and $C_{1-4}$ alkanoyloxy), $C_{1-4}$ alkoxycarbonyl-$C_{1-4}$ alkyl, cyclo-$C_{3-6}$ alkyl, N,N-di-$C_{1-4}$-alkylamino-$C_{1-4}$ alkyl and N,N-di-$C_{1-4}$ alkylcarbamoyl-$C_{1-4}$ alkyl;

$R^5$ represents a group of formula -COQ wherein Q represents hydrogen or $C_{1-4}$ alkyl (optionally substituted by one or more halogen atoms) or phenyl; and

$R^3$ and $R^4$ both represent hydrogen;

and acid addition salts thereof.

The compounds of formula (IIIA) include the subclass wherein $Ar^1$ represents an aryl group substituted by at least one suitable substituent, especially when $Ar^1$ represents substituted phenyl.

Preferred compounds of formula (IIIA) also include those wherein Ar is a substituted phenyl group, in particular where the phenyl group is substituted in the 3- position. Also preferred are compounds of formula (IIIA) wherein $R^1$ is an alkyl group, in particular a methyl group, and compounds of formula (IIIA) wherein $R^5$ is formyl, acetyl or benzoyl.

A particularly preferred compound of formula (IIIA) is where Ar is a phenyl group substituted in the 3- position by a trifluoromethyl group, $R^1$ is methyl, $R^6$ is acetyl and $R^3$ and $R^4$ are both hydrogen, namely, N-methyl-N-[1-(3-trifluoromethylphenyl)-2-pyrazolin-3-yl]acetamide.

Other compounds of formula (IIIA) include the following and their pharmacologically acceptable acid addition salts:-

N-benzyl-N-[1-(3-trifluoromethylphenyl)-2-pyrazolin-3-yl]formamide
N-methyl-N-[1-(3-trifluoromethylphenyl)-2-pyrazolin-3-yl]formamide
N-(2-propenyl)-N-[1-(3-(trifluoromethylphenyl)-2-pyrazolin-3-yl]formamide
N-(2-propynyl)-N-[1-(3-trifluoromethylphenyl)-2-pyrazolin-3-yl]-formamide
N-(n-propyl)-N-[1-(3-trifluoromethylphenyl)-2-pyrazolin-3-yl]-formamide
N,N-diethyl-2-[N-[1-(3-trifluoromethylphenyl)-2-pyrazolin-3-yl]formamido] acetamide
N-[2-(diethylamino)ethyl]-N-[1-(3-trifluoromethylphenyl)-2-pyrazolin-3-yl] formamide
N-(cyclopropylmethyl)-N-[1-(3-trifluoromethylphenyl)-2-pyrazolin-3-yl] formamide
N-(3-phenyl-2-propenyl)-N-[1-(3-trifluoromethylphenyl)-2-pyrazolin-3-yl] formamide

N-(2-propenyl)-N-[1-(3-bromophenyl)-2-pyrazolin-3-yl]formamide
N-(2-propynyl)-N-[1-(3-bromophenyl)-2-pyrazolin-3-yl]formamide
N-methyl-N-[1-(3-bromophenyl)-2-pyrazolin-3-yl]formamide
N-(2-propynyl)-N-[1-(3,5-ditrifluoromethylphenyl)-2-pyrazolin-3-yl]formamide
N-methyl-N-[1-(3,5-ditrifluoromethylphenyl)-2-pyrazolin-3-yl]formamide
N-(2-propynyl)-N-[1-(3-methylthiophenyl)-2-pyrazolin-3-yl]formamide
N-[1-(3-methylthiophenyl)-2-pyrazolin-3-yl]-N-(2-propenyl)acetamide
N-[1-(3-methylthiophenyl)-2-pyrazolin-3-yl]-N-(4-methoxybenzyl)acetamide
N-[1-(4-bromo-3-trifluoromethylphenyl)-2-pyrazolin-3-yl]-N-(4-fluorobenzyl)formamide
N-(4-acetoxybenzyl)-N-[1-(4-bromo-3-trifluoromethyl phenyl) -2-pyrazolin-3-yl]formamide
N-methyl-N-[1-(4-bromo-3-trifluoromethylphenyl)-2-pyrazolin-3-yl]formamide
N-(2-propynyl)-N-[1-(2-pyridyl)-2-pyrazolin-3-yl]formamide
N-(2-propynyl)-[1-(3-quinolyl)-2-pyrazolin-3-yl]formamide
N-(2-propynyl)-N-[1-(3-trifluoromethylphenyl)-2- pyrazolin- 3-yl]acetamide
N-Methyl-N-[1-(3-trifluoromethylphenyl)-2-pyrazolin-3-yl] propionamide
N-ethoxycarbonylmethyl-N-[1-(3-trifluoromethylphenyl)-2-pyrazolin-3-yl]formamide
N-(4-chloro-2-butenyl)-N-[1-(3-trifluoromethylphenyl)-2-pyrazolin-3-yl]formamide
N-(2-propenyl)-N-[1-(3,5-ditrifluoromethyl)-2-pyrazolin-3-yl]formamide
N-methyl-N-[1-(4-chlorophenyl)-2-pyrazolin-3-yl]benzamide
N-(2-ethoxycarbonylethyl)-N-[1-(3-trifluoromethylphenyl)-2-pyrazolin-3-yl] formamide
N-[1-(3-methylthiophenyl)-2-pyrazolin-3-yl]-N-methylacetamide
N-methyl-N-[1-(3-bromophenyl)-2-pyrazolin-3-yl]acetamide
N-methyl-N-[1-(3-bromophenyl)-2-pyrazolin-3-yl]acetamide
N-methyl-N-[1-(3-quinolyl)-2-pyrazolin-3-yl]acetamide
N-propyl-N-[1-(3-trifluoromethylphenyl)-2-pyrazolin-3-yl]acetamide
N-ethyl-N-[1-(3-trifluoromethylphenyl)-2-pyrazolin-3-yl]acetamide
N-methyl-N-[1-(3-nitrophenyl)-2-pyrazolin-3-yl]acetamide
N-methyl-N-[1-(4-bromo-3-trifluoromethylphenyl)-2-pyrazolin-3-yl]acetamide
N-methyl-N-[1-(4-methylthiophenyl)-2-pyrazolin-3-yl]acetamide
N-(2-propenyl)-N-[1-(3-methylthiophenyl)-2-pyrazolin-3-yl]acetamide
N-methyl-N-[1-(3-trifluoromethylphenyl)-2-pyrazolin-3-yl]trifluoroacetamide

Non-steroidal (or aspirin-like) anti-inflammatory drugs are known in the art. This class of drugs inhibits the formation of prostaglandins by inhibiting the cyclooxygenase pathway of arachidonic acid metabolism, thus reducing erythema, oedema and pain production associated with prostaglandins, leading to good symptomatic relief. Aspirin-like drugs are not however effective against the leucocyte-mediated aspects of chronic inflammatory disease, since they do not inhibit leukotriene formation. In contrast to non-steroid anti-inflammatory drugs, corticosteroidal anti-inflammatory drugs indirectly inhibit the formation of both prostaglandins and leucotrienes, which could, in part, account for their superior therapeutic effect when compared to aspirin-like drugs. Steroid treatment is, however, associated with serious local or systemic side effects and prolonged administration is contra-indicated in many inflammatory conditions.

The compounds of formula (IIIA) fulfill the need for a non-steroid anti-inflammatory compound which inhibits the synthesis of leukotrienes and prostaglandins and which is free from steroid related toxicity. They have also been shown to be more stable than closely related compounds known in the art, such as are disclosed in European Specification 55 418.

When used in medicine, the acid addition salts (where such exist) of a compound of formula (IIIA) should be both pharmacologically and pharmaceutically acceptable, but non-acceptable salts may conveniently be used to prepare the bases of such acceptable salts and are not excluded from the scope of this invention. Acceptable salts may be derived from organic acids, particularly dicarboxylic acids. Such pharmacologically and pharmaceutically acceptable salts include those prepared from the following acids: hydrochloric, hydrobromic, sulphuric, nitric, phosphoric, oxalic, fumaric, maleic, glycolic, salicyclic, succinic, toluene-p-sulphonic, tartaric, acetic, citric, methane sulphonic, formic, benzoic, malonic, naphthalene-2-sulphonic and benzene sulphonic.

The compounds of formula (IIIA) may be prepared by stage (i) of the aforementioned process of the present invention or by any method analogous to those known in the art, for example, one method comprises acylation or aroylation of a compound of formula (V)

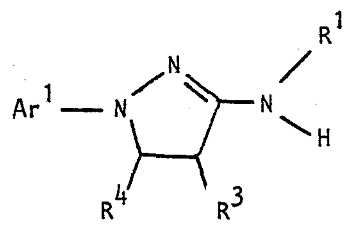

(V)

wherein,

Ar, $R^1$, $R^3$ and $R^4$ are as defined for formula (IIIA), with a suitable acid halide, acid anhydride or mixed anhydride. The reaction may be effected in an inert solvent, for example a halogenated hydrocarbon, at a temperature in the range of 0-100$^o$, and optionally in the presence of a base, for example, an organic tertiary amine, or inorganic base or an excess of the pyrazoline of formula (V).

The compounds of formula (IIIA) may be used in the relief of rheumatoid arthritis, rheumatoid spondylitis, osteoarthritis, gouty arthritis and other arthritic conditions, inflamed joints, psoriasis, eczema, inflammatory eye conditions including conjuctivitis, pyresis and other conditions associated with inflammation and pain. Such other conditions associated with inflammation include the reduction of tissue necrosis in chronic inflammation, the suppression of tissue rejection following transplant surgery and ulcerative colitis.

The compounds of formula (IIIA) may also be used in the treatment or prophylaxis of allergic conditions and airway inflammatory conditions such as asthma and of asthma having a non-allergic origin and bronchitis. The compounds may also be useful as antispasmogenic agents.

Thus the present invention further provides a compound of formula (IIIA) or a pharmacologically acceptable acid addition salt thereof for use in a method of inhibiting the lipoxygenase and cyclooxygenase pathways of the arachidonic acid metabolism in a mammal such as man. The invention also provides a compound of formula (IIIA) or a pharmacologically acceptable acid addition salt thereof for use in a method of prophylaxis or treatment, by therapy, of a mammal such as man, especially for the therapy of any disease or condition specified herein.

The amount required of a compound of formula (IIIA) (hereinafter referred to as the active ingredient) for therapeutic effect will, of course, vary both with the particular compound, the route of administration and the mammal under treatment. A suitable dose of a compound of formula (IIIA) for a mammal

suffering from an inflammatory, painful or pyretic condition as defined hereinbefore is 0.1 µg-500mg of base per kilogram body weight. In the case of systemic administration, the does may be in the range 0.5 to 500 mg of base per kilogram bodyweight, the most preferred dosage being 0.5 to 50 mg/kg of mammal bodyweight for example 5 to 25 mg/kg; administered two or three times daily. In the case of topical administration, eg. to the skin or eye, a suitable dose may be in the rang 1ng -100µg of base per kilogram, typically about 0.1 µg/kg.

In the case of the treatment or prophylaxis of inflammatory airway conditions, a suitable anti-asthmatic dose of a compound of formula (IIIA) is 1 mg to 10 mg of base per kilogram, the most preferred dosage being 1 mg to 5 mg/kg of mammal bodyweight, for example from 1 to 2 mg/kg.

While it is possible for an active ingredient to be administered alone, it is preferable to present it as a pharmaceutical formulation comprising a compound of formula (IIIA) or a pharmacologically acceptable acid addition salt thereof, and a pharmaceutically acceptable carrier therefor. Such formulations constitute a further feature of the present invention. Conveniently, the active ingredient comprises from 0.1% to 99.9% by weight of the formulation. Conveniently, unit doses of a formulation contain between 0.1 mg and 1 g of the active ingredient. For topical administration, the active ingredient preferably comprises from 1% to 2% by weight of the formulation but the active ingredient may comprise as much as 10% w/w. Formulations suitable for nasal or buccal administration, (such as self-propelling powder dispensing formulations described hereinafter), may comprise 0.1 to 20% w/w, for example 2% w/w of active ingredient.

The formulations, both for veterinary and for human medical use, of the present invention comprise an active ingredient in association with a pharmaceutically acceptable carrier therefor and optionally other therapeutic ingredient(s). The carrier(s) must be 'acceptable' in the sense of being compatible with the other ingredients of the formulations and not deleterious to the recipient thereof.

The formulations include those in a form suitable for oral, ophthalmic, rectal, parenteral (including subcutaneous, intramuscular and intravenous), intra-articular, topical, nasal or buccal administration.

The formulations may conveniently be presented in unit dosage form and may

be prepared by any of the methods well known in the art of pharmacy. All methods include the step of bringing the active ingredient into association with the carrier which constitutes one or more accessory ingredients. In general, the formulations are prepared by uniformly and intimately bringing the active ingredient into association with a liquid carrier or a finely divided solid carrier or both, and then, if necessary, shaping the product into the desired formulation.

Formulations of the present invention suitable for oral administration may be in the form of discrete units such as capsules, cachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion. The active ingredient may also be in the form of a bolus, electuary or paste.

A tablet may be made by compressing or moulding the active ingredient optionally with one or more accessory ingredients. Compressed tablets may be prepared by compressing, in a suitable machine, the active ingredient in a free-flowing form such as a powder or granules, optionally mixed with a binder, lubricant, inert diluent, surface active or dispersing agent. Moulded tablets may be made by moulding, in a suitable machine, a mixture of the powdered active ingredient and a suitable carrier moistened with an inert liquid diluent.

Formulations for rectal administration may be in the form of a suppository incorporating the active ingredient and a carrier such as cocoa butter, or in the form of an enema.

Formulations suitable for parenteral administration conveniently comprise a sterile aqueous preparation of the active ingredient which is preferably isotonic with the blood of the recipient.

Formulations suitable for intra-articular administration may be in the form of a sterile aqueous preparation of the active ingredient which may be in microcrystalline form, for example, in the form of an aqueous microcrystalline suspension. Liposomal formulations or biodegradable polymer systems may also be used to present the active ingredient for both intra-articular and ophthalmic administration.

Formulations suitable for topical administration include liquid or semi-liquid preparations such as liniments, lotions, applications; oil-in-water or

water-in-oil emulsions such as creams, ointments or pastes; or solutions or suspensions such as drops. For example, for ophthalmic administration, the active ingredient may be presented in the form of aqueous eye drops as, for example, a 0.1 - 1.0% solution.

Formulations suitable for administration to the nose or buccal cavity include powder, self-propelling and spray formulations such as aerosols and atomizers. The formulations, when dispersed, preferably have a particle size in the range of 10 to 200μ.

Formulations of the present invention may also be in the form of an aqueous or dilute alcoholic solution, optionally a sterile solution, of the active ingredient for use in a nebuliser or atomiser, wherein an accelerated air steam is used to produce a fine mist consisting of small droplets of the solution. Such formulations usually contain a flavouring agent such as saccharin sodium and a volatile oil. A buffering agent and a surface active agent may also be included in such a formulation which should also contain a preservative such as methylhydroxybenzoate.

Other formulations suitable for nasal administration include a coarse powder having a particle size of 20 to 500 microns which is administered in the manner in which snuff is taken i.e. by rapid inhalation through the nasal passage from a container of the powder held close up to the nose.

In addition to the aforementioned ingredients, the formulations of this invention may include one or more additional ingredients such as diluents, buffers, flavouring agents, binders, surface active agents, thickeners, lubricants preservatives eg. methylhydroxybenzoate (including anti-oxidants), emulsifying agents and the like.

Any other therapeutic ingredient may comprise one or more of the following: antibiotic, anti-fungal and anti-viral agents.

Within the scope of the present invention are all novel features described herein, including:-

(a) a process for the preparation of a compound of formula (IIIA) or an acid addition salt thereof;

(b) a compound of formula (IIIA) or an acid addition on salt thereof;

(c) a pharmaceutical formulation comprising a non-toxic, effective arachidonic acid oxygenation inhibitory amount of a compound of formula (IIIA) or a pharmacologically acceptable salt thereof and a pharmaceutically acceptable carrier therefor;

(d) a method for preparing such formulations;

(e) a method for the inhibition of the lipoxygenase and cyclooxygenase pathways of arachidonic acid metabolism comprising the administration of a non-toxic, effective, inhibitory amount of a compound of formula (IIIA) or an acid addition salt thereof;

(f) a method for the prophylaxis or treatment of inflammation in a mammal, including man, comprising the administration to said mammal of a non-toxic, effective anti-inflammatory amount of a compound of formula (IIIA) or a pharmacologically acceptable acid addition salt thereof;

(g) a method for the prophylaxis or treatment of pain in a mammal, including man, comprising the administration to said mammal of a non-toxic effective analgesic amount of a compound of formula (IIIA) or a pharmacologically acceptable acid addition salt thereof;

(h) a method for the prophylaxis or treatment of pyresis in a mammal, including man, comprising the administration to said mammal of a non-toxic, effective anti-pyretic amount of a compound of formula (IIIA) or a pharmacologically acceptable acid addition salt thereof;

(i) a method for the prophylaxis or treatment of asthma in a mammal, including man, comprising administration to said mammal of a non-toxic, effective, anti-asthmatic amount of a compound of formula (IIIA) or a pharmacologically acceptable acid addition salt thereof;

(j) a compound of formula (IIIA) or a pharmacologically acceptable acid addition salt thereof for use in medicine in the inhibition of the lipoxygenase or cyclooxygenase pathways of arachidonic acid metabolism;

(k) a novel compound of formula (I) or an acid addition salt thereof;

(l) a method for preparing a compound of formula (I);

(m) a pharmaceutical formulation comprising a non-toxic, effective arachidonic acid oxygenation inhibitory amount of a compound of formula (I) or a pharmacologically acceptable acid addition salt thereof and a pharmaceutically acceptable carrier therefor;

(n) a method for preparing such formulations;

(o) a method for the inhibition of the lipoxygenase and cyclooxygenase pathways of arachidonic acid metabolism comprising the administration of a non-toxic, effective, inhibitory amount of a compound of formula (I) or an acid addition salt thereof;

(p) a method for the prophylaxis or treatment of inflammation in a mammal, including man, comprising the administration to said mammal of a non-toxic, effective anti-inflammatory amount of a compound of formula (I) or a pharmacologically acceptable acid addition salt thereof;

(q) a method for the prophylaxis or treatment of pain in a mammal, including man, comprising the administration to said mammal of a non-toxic effective analgesic amount of a compound of formula (I) or a pharmacologically acceptable acid addition salt thereof;

(r) a method for the prophylaxis or treatment of asthma in a mammal, including man, comprising administration to said mammal of a non-toxic, effective, anti-asthmatic amount of a compound of formula (I) or a pharmacologically acceptable acid addition salt thereof;

(s) a method for the prophylaxis or treatment of pyresis in a mammal, including man, comprising the administration to said mammal of a non-toxic, effective anti-pyretic amount of a compound of formula (I) or a pharmacologically acceptable acid addition salt thereof;

(t) a compound of formula (I) or a pharmacologically acceptable acid addition salt thereof for use in medicine in the inhibition of the lipoxygenase and cyclo-oxygenase pathways of arachidonic acid metabolism; and

(u) Any novel compound of formula (II) (or an acid addition salt thereof, especially a pharmacologically acceptable acid addtion salt), a process for its preparation, a pharmaceutical formulation containing such a compound or salt, and its use in medicine.

The following examples are provided by way of an illustration of the present invention and should in no way be construed as a limitation thereof. All temperatures are indicated in degrees Celsius.

Example 1 : Preparation of 3-benzylamino-1-(3-trifluoromethylphenyl)-2-pyrazoline hydrochloride

A. Preparation of N-[1-(3-trifluoromethylphenyl)-2-pyrazolin-3-yl]formamide

Acetic anhydride (32.5ml) was added over 30 minutes to 3-amino-1-(3-trifluoromethylphenyl)-2-pyrazoline (25g) in 98% formic acid (100ml) at 55-60°. The mixture was stirred at 55-60° and then cooled to ambient temperature. Dropwise addition of water (125ml) precipitated N-[1-(3-trifluoromethylphenyl)-2-pyrazolin-3-yl]formamide, m.p. 126-127° after recrystallisation from toluene.

B. Preparation of N-benzyl-N-[1-(3-trifluoromethylphenyl)-2-pyrazolin-3-yl] formamide

Sodium hydride (50% dispersion in oil, 0.48 g) was added to a solution of N-[1-(3-trifluoromethylphenyl)-2-pyrazolin-3-yl]formamide (2.57 g) in N,N-dimethylformamide (30 ml) under an atmosphere of nitrogen, and the solution was stirred until the evolution of hydrogen had ceased. Benzyl chloride (1.27 ml) and sodium iodide (0.15 g) were added, and the mixture was stirred for 2 hours at room temperature and 15 minutes at 100° ( to pH 7) then quenched with ice-water to furnish N-benzyl-N-[1-(3-trifluoromethylphenyl)-2-pyrazolin-3-yl] formamide (2.18 g), m.p. 125-127° after recrystallisation from ethanol.

C. Preparation of 3-benzylamino-1-(3-trifluoromethylphenyl)-2-pyrazoline hydrochloride

A mixture of N-benzyl-N-[1-(3-trifluoromethylphenyl)-2-pyrazolin-3-yl-]formamide (0.347 g), ethanol (5 ml), and hydrazine (0.035 ml) was heated under

reflux for 12 hours, allowed to cool, and evaporated in vacuo. The oily residue was partitioned between diethyl ether and 2N hydrochloric acid to precipitate 3-benzylamino-1-(3-trifluoromethylphenyl)-2-pyrazoline hydrochloride (0.24 g), m.p. 185-190°.

Example 2 : Preparation of 3-methylamino-1-(3-trifluoromethylphenyl)-2-pyrazoline

A. Preparation of N-methyl-N-[1-(3-trifluoromethylphenyl)-2-pyrazolin-3-yl] formamide

50% Sodium hydride dispersion in mineral oil (0.48g) was added in portions to a solution of N-[1-(3-trifluoromethylphenyl)-2-pyrazolin-3-yl]formamide (2.57) g in N,N-dimethylformamide (30 ml), and the mixture stirred until the evolution of hydrogen had ceased. Iodomethane (0.75ml) was added, and the solution stirred at ambient temperature until the pH reached ca.7. The mixture was quenched with ice-water to yield N-methyl-N-[1-(3-trifluoromethylphenyl)-2-pyrazolin-3-yl]-formamide, 2.65 g, m.p. 121-123° after recrystallisation from ethanol.

B. Preparation of 3-methylamino-1-(3-trifluoromethylphenyl)-2-pyrazoline

The title compound was prepared by the following alternative routes (a)-(c):-

(a) A mixture of N-methyl-N-[1-(3-trifluoromethylphenyl)-2-pyrazolin-3-yl]-formamide (1.08g), and hydrazine (0.15ml) in ethanol (20ml) was heated under reflux for 5 days, and then evaporated in vacuo. The residue was recrystallised from light petroleum (60-80°) to yield 3-methylamino-1-(3-tri fluoromethylphenyl)-2-pyrazoline m.p. 90-92°.

(b) A mixture of N-methyl-N-[1-(3-trifluoromethylphenyl)-2-pyrazolin-3-yl]-formamide (0.256g, prepared according to example 2A), 1.0N sodium hydroxide (1.1ml) and ethanol (sufficient to give a homogenous solution, ca 10ml) was stirred for 1 hour at ambient temperature. Removal of solvent in vacuo, followed by isolation with ether, afforded 3-methylamino-1-(3-trifluoromethylphenyl)- 2-pyrazoline, identical with an authentic sample.

(c) A mixture of N-methyl-N-[1-(3-trifluoromethylphenyl)-2-pyrazolin-3-yl]-formamide (0.092g, prepared according to example 2A), and 0.5ml of a mixture of concentrated hydrochloric acid (5.5ml) and methanol (60ml) was stirred at ambient temperature for 48 hours. Evaporation of solvent in vacuo yielded 3-methylamino-1-(3-trifluoromethylphenyl)-2-pyrazoline hydrochloride, identical with an authentic sample.

Example 3: Preparation of 3-(2-propenylamino)-1-(3-trifluoromethylphenyl)-2--pyrazoline

A. Preparation of N-(2-propenyl)-N-[1-(3-(trifluoromethylphenyl)-2-pyrazolin--3-yl]formamide

By a procedure analogous to that of example 2A, N-[1-(3-trifluoromethylphenyl)- 2-pyrazolin-3-yl]formamide was treated with allyl bromide, to yield after recrystallisation from light petroleum (60-80 $^{o}$) N-(2-propenyl)-N-[1-(3-trifluoromethylphenyl)-2-pyrazolin-3-yl]formamide,m.p. 78-79$^{o}$.

B. Preparation of 3-(2-propenylamino)-1-(3-trifluoromethylphenyl)-2-pyrazoline

By a procedure analogous to that described in Example 2B(b), N-(2-propenyl)--N-[1-(3-trifluoromethylphenyl)-2-pyrazolin-3-yl]formamide (1 g) was treated with 4 ml of 1.0N sodium hydroxide solution and ca 18ml of ethanol, to yield 3-(2-propenylamino)-1-(3-trifluoromethylphenyl) -2-pyrazoline, the hydrochloride salt 0.84 g m.p. 144-146$^{o}$.

Example 4: Preparation of 3-(2-propynyl)-1-(3-trifluoromethylphenyl)-2-pyrazoline

A. Preparation of N-(2-propynyl)-N-[1-(3-trifluoromethylphenyl)-2-pyrazolin-3-yl]-formamide

By a procedure analogous to that of example 2A, N-[1-(3-trifluoro methylphenyl)-2-pyrazolin-3-yl]formamide was treated with propargyl bromide in toluene to yield, after recrystallisation from ethanol, N-(2-propynyl)-N-[1-(3-trifluoromethylphenyl)-2-pyrazolin-3-yl]formamide 0.9 g, m.p. 113-115$^{o}$.

B. Preparation of 3-(2-propynylamino)-1-(3-trifluoromethylphenyl) -2-pyrazoline

By a procedure analogous to that of example 2B(b), N-(2-propynyl) -N-[1-(3-trifluoromethylphenyl)-2-pyrazolin-3-yl]formamide (0.6g) was stirred with 2.1ml of 1.0N sodium hydroxide solution and ca 25ml of ethanol, for 0.5 hours at ambient temperature to yield, after recrystallisation from light petroleum (60-80$^o$), 3-(2-propynylamino)-1-(3-trifluoromethylphenyl)-2-pyrazoline, 0.3 g, m.p. 68-69$^o$.

Example 5: Preparation of 3-(n-propylamino)-1-(3-trifluoromethylphenyl)-2-pyrazoline

A. Preparation of N-(n-propyl)-N-[1-(3-trifluoromethylphenyl)-2-pyrazolin-3-yl]-formamide

By a procedure analogous to that of example 2A, N-[1-(3-trifluoro methylphenyl)- 2-pyrazolin-3-yl]formamide was treated with n-propyl bromide in N,N-dimethylformamide to afford, after recrystallisation from light petroleum (60-80$^o$), N-n-propyl-N-[1-(3-trifluoromethylphenyl)-2-pyrazolin-3-yl]formamide,4.4 g m.p. 89-91$^o$.

B. Preparation of 3-(n-propylamino)-1-(3-trifluoromethylphenyl)-2-pyrazoline

By a procedure analogous to that of example 2B(b), N-(n-propyl)-N-[1-(3-trifluoromethylphenyl)-2-pyrazolin-3-yl]formamide was stirred with 12.25ml of 1.0N sodium hydroxide solution and ca 85ml of ethanol for 48 hours at ambient temperature to yield, 3-(n-propylamino)-1-(3-trifluoromethylphenyl)-2-pyrazoline. The hydrochloride salt (1.8 g), had m.p. 150-152$^o$.

Example 6: Preparation of N,N-diethyl-3-[1-(3-trifluoromethylphenyl)-2-pyrazolin-3-yl]glycinamide

A. Preparation of N,N-diethyl-2-[N-[1-(3-trifluoromethylphenyl)-2-pyrazolin-3-yl]formamido]acetamide

By a procedure analogous to that of example 2A, N-[1-(3-trifluoro methylphenyl)- 2-pyrazolin-3-yl]formamide was treated with 2-bromo-N,N-diethylacetamide to yield, after recrystallisation from ethanol, N,N-diethyl-2-[N-[1-(3-trifluoromethylphenyl)-2-pyrazolin-3-yl]formamido]-acetamide, m.p. 115-117$^o$.

B. Preparation of N,N-diethyl-3-[1-(3-trifluoromethylphenyl)-2-pyrazolin-3-yl] glycinamide

By a procedure analogous to that of example 2B(a), N,N-diethyl-2-[N-[1-(3-trifluoromethylphenyl)-2-pyrazolin-3-yl]formamido]acetamide was heated under reflux with hydrazine in ethanol for 4 days, to yield, after recrystallisation from ethanol, N,N-diethyl-3-[1-(3-trifluoromethylphenyl)-2-pyrazolin-3-yl]glycinamide, 0.4 g m.p. 125-126°.

Example 7 : Preparation of 3-[(2-diethylamino)ethyl]amino]-1-(3-trifluoromethylphenyl)-2-pyrazoline

A. Preparation of N-[2-(diethylamino)ethyl]-N-[1-(3-trifluoromethylphenyl)-2-pyrazolin-3-yl]formamide

By a procedure analogous to that of example 2A, N-[1-(3-trifluoromethylphenyl)-2-pyrazolin-3-yl]formamide was treated with 2-(diethylamino)ethylchloride, to form, as an oil, N-[2-(diethylamino)ethyl]-N-[1-(3-trifluoromethylphenyl)- 2-pyrazolin-3-yl]formamide.

B. Preparation of 3-[(2-diethylamino)ethyl]amino]-1-(3-trifluoromethylphenyl)-2-pyrazoline

By a procedure analogous to example 2B(b), N-[2-(diethylamino)ethyl]-N-[1-(3-trifluoromethylphenyl)-2-pyrazolin-3-yl]formamide was treated with 1.0N sodium hydroxide solution in ethanol to yield 3-[(2-diethylamino)ethyl]amino]-1-(3-trifluoromethylphenyl)-2-pyrazoline. The di-hydrobromide salt (0.9 g) had m.p. 183-185°.

Example 8 : Preparation of 3-[(cyclopropylmethyl)amino]-1-(3-trifluoromethylphenyl)-2-pyrazoline

A. Preparation of N-(cyclopropylmethyl)-N-[1-(3-trifluoromethylphenyl)-2-pyrazolin-3-yl]formamide

By a procedure analogous to that of example 2A, N-[1-(3-trifluoromethylphenyl)-2-pyrazolin-3-yl]formamide was treated with cyclopropylmethyl bromide to yield, after recrystallisation from ethanol, N-(cyclopropylmethyl)-N-[1-(3-trifluoromethylphenyl)-2-pyrazolin-3-yl]formamide m.p. 111-113°.

B. Preparation of 3-[(cyclopropylmethyl)amino]-1-(3-trifluoromethylphenyl)-2-pyrazoline

By a procedure analogous to example 2B(b), N-(cyclopropylmethyl)-N-[1-(3-trifluoromethylphenyl)-2-pyrazolin-3-yl]formamide was treated with 1.0N sodium hydroxide solution in ethanol, for 24 hours at ambient temperature and then 12 hours at $50^{0}$ to yield, after recrystallisation from light petroleum ($60-80^{0}$), 3-[(cyclopropylmethyl)amino]-1-(3-trifluoromethylphenyl)-2-pyrazoline, m.p. $73-75^{0}$.

Example 9 : Preparation of 3-(cinnamylamino)-1-(3-trifluoromethylphenyl)-2-pyrazoline

A. Preparation of N-(cinnamyl)-N-[1-(3-trifluoromethylphenyl)-2-pyrazolin-3-yl]formamide

By a procedure analogous to that of example 2A, N-[1-(3-trifluoromethylphenyl)-2-pyrazolin-3-yl]formamide was treated with cinnamyl chloride to afford, after recrystallisation from ethanol, N-(cinnamyl)-N-[ 1-(3-trifluoromethylphenyl)-2-pyrazolin-3-yl]formamide, m.p. $114-115^{0}$.

B. Preparation of 3-(cinnamylamino)-1-(3-trifluoromethylphenyl)-2-pyrazoline

By a procedure analogous to example 2B(b), N-(3-phenyl-2-propenyl)-N-[1-(3-trifluoromethylphenyl)-2-pyrazolin-3-yl]formamide was stirred with 1.0N sodium hydroxide solution in ethanol for 12 hours at ambient temperature, to yield, after recrystallisation from light petroleum ($60-80^{0}$), 3-(cinnamylamino)-1-(3-trifluoromethylphenyl)-2-pyrazoline, m.p. $71-73^{0}$.

Examples 10-12

N-[1-(3-bromophenyl)-2-pyrazolin-3-yl]formamide, m.p. $152.5-153.5^{0}C$ was prepared in a manner analogous to that described in Example 1A. The latter compound was converted to the following compounds (A) of formula (III) in a manner analogous to that described in Example 1B, which were in turn converted to the corresponding compounds (B) of formula (I) by methods analogous to that of Example 2B(b):

10) A. N-(2-propenyl)-N-[1-(3-bromophenyl)-2-pyrazolin-3-yl]formamide, m.p.90.5-91°C.

B. 3-(2-propenylamino)-1-(3-bromophenyl)-2-pyrazoline hydrochloride, m.p. 140.5-142°C.

11) A. N-(2-propynyl)-N-[1-(3-bromophenyl)-2-pyrazolin-3-yl]formamide, m.p.160.5-161°C.

B. 3-(2-propynylamino)-1-(3-bromophenyl)-2-pyrazoline. p-TsOH, m.p. 153.5-154.5°C.

12) A. N-methyl-N-[1-(3-bromophenyl)-2-pyrazolin-3-yl]formamide, m.p.130-130.5°C.

B. 3-methylamino-1-(3-bromophenyl)-2-pyrazoline hydrochloride, m.p. 203-205°C.

Examples 13 and 14

N-[1-(3,5-ditrifluoromethyl)-2-pyrazolin-3-yl]formamide, m.p. 190-191°C was prepared in a manner analogous to that described in Example 1A. The latter compound was converted to the following compounds (A) of formula (III) in a manner analogous to that described in Example 1B, which were in turn converted to the corresponding compounds (B) of formula (I) by methods analogous to that of Example 2B(b):-

13) A. N-(2-propynyl)-N-[1-(3,5-ditrifluoromethylphenyl)-2-pyrazolin-3-yl]-formamide, m.p.124-125°C.

B. 3-(2-propynylamino)-1-(3,5-ditrifluoromethylphenyl)-2-pyrazoline, m.p. 117.5 - 118.5°C.

14) A. N-methyl-N-[1-(3,5-ditrifluoromethylphenyl)-2-pyrazolin-3-yl]formamide, m.p.189-190.5°C.

B. 3-methylamino-1-(3,5-ditrifluoromethylphenyl)-2-pyrazoline, m.p. 98- 99°C.

Example 15: Preparation of 3-(2-propynylamino)-1-(3-methylthiophenyl)-2-pyrazoline hydrochloride

A. Preparation of N-[1-(3-Methylthiophenyl)-2-pyrazolin-3-yl]formamide

Acetic anhydride (9ml) was added dropwise to a solution of 3-amino-1-(3-methylthiophenyl)-2-pyrazoline (6g) in 98-100% formic acid (30 ml) at $60^0$. The mixture was stirred for 0.5 hr at $60^0$, cooled to room temperature and the crude product was precipitated by the dropwise addition of water. The crude product was isolated, dried and recrystallised from benzene to yield N-[1-(3-methylthiophenyl)-2-pyrazolin-3-yl]formamide, 2.67g, m.p. 125-$127^0$.

B. Preparation of N-(2-propynyl)-N-[1-(3-methylthiophenyl)-2-pyrazolin-3-yl] formamide

By a procedure analogous to that of example 1B, N-[1-(3-methylthiophenyl)-2-pyrazolin-3-yl]formamide was treated with propargyl bromide in toluene to yield N-(2-propynyl)-N-[1-(3-methylthiophenyl)-2-pyrazolin-3-yl]formamide m.p. 119-$122^0$, after a wash with light petroleum (60-$80^0$).

C. Preparation of 3-(2-propynylamino)-1-(3-methylthiophenyl)-2-pyrazoline hydrochloride

The title compound was prepared from the title compound of Example 15B, in a manner analogous to that described in Example 2B(b), m.p. $135^0$C (decomp).

Example 16: Preparation of 1-(3-methylthiophenyl)-3-(2-propenyl)-2-pyrazoline hydrochloride

A. Preparation of N-[1-(3-methylthiophenyl)-2-pyrazolin-3-yl]-N-(2-propenyl)acetamide

A 50% dispersion of sodium hydride in mineral oil (0.39g) was added to N-[1-(3-methylthiophenyl)-2-pyrazolin-3-yl]acetamide (1.9g) in N,N-dimethylformamide (30 ml), and the mixture was stirred until the evolution of hydrogen ceased. Allyl bromide (1.1 ml) was added, and the mixture was stirred for 1 hr at the ambient temperature (to pH ca. 7), then quenched with ice-water to furnish N-[1-(3-methylthiophenyl)-2-pyrazolin-3-yl]-N- (2-propenyl)acetamide, m.p. 51-$52.5^0$ (from methanol-water).

B. Preparation of 1-(3-methylthiophenyl)-3-(2-propenyl)-2-pyrazoline hydrochloride

A solution of N-[1-(3-methylthiophenyl)-2-pyrazolin-3-yl]-N-(2-propenyl) acetamide (1.7g) in 1.0 N sodium hydroxide (6.8 ml) and ethanol (ca. 90 ml) was

stirred for 2hr at room temperature. Evaporation and isolation with ether afforded 1-(3-methylthiophenyl)-3-(2-propenyl)-2-pyrazoline as an oil. The hydrochloride salt had m.p. 132-134$^{o}$C (from methanol-ether).

Example 17: Preparation of 3-(4-methoxybenzylamino)-1-(3-methylthiophenyl)-2-pyrazoline

A. Preparation of N-[1-(3-methylthiophenyl)-2-pyrazolin-3-yl]-N-(4-methoxybenzyl)acetamide

By following the procedure of Example 16A but using 4-methoxybenzyl chloride in place of allyl bromide, there was obtained N-[1-(3-methylthiophenyl)-2-pyrazolin-3-yl]-N-(4-methoxybenzyl)acetamide as an oil which was purified by chromatography over silica gel (elution with chloroform).

B. Preparation of 3-(4-methoxybenzylamino)-1-(3-methylthiophenyl)-2-pyrazoline

By following the procedure of Example 16B but using N-[1-(3-methylthiophenyl)-2-pyrazolin-3-yl]-N-(4-methoxybenzyl)acetamide as the starting material, there was obtained 3-(4-methoxybenzylamino)-1-(3-methylthiophenyl)-2-pyrazoline, m.p. 82-84$^{o}$C (from ethanol-water).

Example 18: Preparation of 3-(4-fluorobenzylamino)-1-(4-bromo-3-trifluoro methylphenyl)pyrazoline hydrochloride

A. Preparation of N-[1-(4-bromo-3-trifluoromethylphenyl)-2-pyrazolin-3-yl]-formamide

By a procedure analogous to that of example 1A, 3-amino-1-(4-bromo-3-trifluoromethylphenyl)-2-pyrazoline was treated with acetic anhydride and formic acid to yield, after recrystallisation from toluene, N-[1-(4-bromo-3-trifluoromethylphenyl)-2-pyrazolin-3-yl]formamide, m.p. 156-158$^{o}$.

B. Preparation of N-[1-(4-bromo-3-trifluoromethylphenyl)-2-pyrazolin-3-yl]-N-(4-fluorobenzyl)formamide

By a procedure analogous to that of example 1B, N-[1-(4-bromo-3-trifluoromethylphenyl)-2-pyrazolin-3-yl]formamide, was treated with 4-fluorobenzyl chloride

in N,N-dimethylformamide in the presence of sodium iodide as a catalyst, to yield, after recrystallisation from ethyl acetate-light petroleum (60-80°), N-[1-(4-bromo-3-trifluoromethylphenyl)-2-pyrazolin-3-yl]-N-(4-fluorobenzyl)formamide, m.p. 160.5-162.5°.

C. 3-(4-fluorobenzylamino)-1-(4-bromo-3-trifluoromethylphenyl)-2-pyrazoline hydrochloride

The product from Example 18B was converted to the title compound by a method analogous to that described in Example 2B(b), m.p. 160-162°C.

Examples 19 and 20

The title compound of Example 18A was converted to the following compounds (A) of formula (III) in a manner analogous to that described in Example 1B, which were in turn converted to the corresponding compounds (B) of formula (I) by methods analogous to that of Example 2B(b):-

19) A. N-(4-acetoxybenzyl)-N-[1-(4-bromo-3-trifluoromethyl phenyl) -2-pyrazolin-3-yl]formamide, m.p. 159-169°C (decomp).

B. 3-(4-hydroxybenzyl)-1-(4-bromo-3-trifluoromethylphenyl)-2-pyrazoline, m.p. 120.5-122°C (in this Example, the 4-acetoxy group was hydrolysed to the 4-hydroxy).

20) A. N-methyl-N-[1-(4-bromo-3-trifluoromethylphenyl)-2-pyrazolin-3-yl] formamide, m.p.166.5-168.5°C.

B. 3-methylamino-1-(4-bromo-3-trifluoromethylphenyl)-2-pyrazoline, m.p. 120.5-122°C.

Example 21

The following compound (A) of formula (III) was made in a manner analogous to that described in Example 1A and 1B, and then converted to the compound (B) of formula (I) in a manner analogous to that described in Example 1C.

A. N-(2-propynyl)-N-[1-(2-pyridyl)-2-pyrazolin-3-yl]formamide,m.p.140.8-141.2°C.

B. 3-(2-propynylamino)-1-(2-pyridyl)-2-pyrazoline, m.p. 143-144.5°C.

Example 22

The following compound (A) of formula (III) was made in a manner analogous to that described in Example 1A and 1B, and then converted to the compound (B) of formula (I) in a manner analogous to that described in Example 2B(b).

A. N-(2-propynyl)-[1-(3-quinolyl)-2-pyrazolin-3-yl]formamide, m.p. 200- 202$^{\circ}$C.

B. 3-(2-propynyl)-1-(3-quinolyl)-2-pyrazoline, m.p.162.8$^{\circ}$C.

Example 23

The following compound (A) of formula (III) was made in a manner analogous to that described in Example 1A and 1B, and then converted to the compound (B) of formula (I) in a manner analogous to that described in Example 1C.

A. N-(2-propynyl)-N-(1-phenyl-2-pyrazolin-3-yl)formamide,m.p.169$^{\circ}$C.
B. 3-(2-propynylamino)-1-phenyl-2-pyrazoline, m.p. 99-100$^{\circ}$C.

Example 24

The following compound (A) of formula (III) was made in a manner analogous to that described in Example 1A and 1B, and then converted to the compound (B) of formula (I) in a manner analogous to that described in Example 2B(b).

A. N-(4-methoxybenzyl)-N-(1-phenyl-2-pyrazolin-3-yl)formamide,m.p. 174-176$^{\circ}$C.
B. 3-(4-methoxybenzylamino)-1-phenyl-2-pyrazoline, m.p. 111-111.5$^{\circ}$C.

Example 25 : Preparation of 3-(methylamino)-1-phenyl-2-pyrazoline hydrochloride.

A. Preparation of N-methyl-N-(1-phenyl-2-pyrazolin-3-yl)formamide

By a procedure analogous to that of example 2A, N-(1-phenyl-2-pyrazolin-3-yl)formamide was treated with iodomethane in N,N-dimethylformamide, to yield, after recrystallisation from ethyl acetate-light petroleum (60-80$^{\circ}$), N-methyl-N-(1-phenyl-2-pyrazolin-3-yl)-formamide, 4.3 g, m.p. 141-143$^{\circ}$.

B. Preparation of 3-(methylamino)-1-phenyl-2-pyrazoline hydrochloride

By a procedure analogous to that of example 2B(c), N-methyl-N-(1-phenyl-2-pyrazolin-3-yl)formamide (2.8 g) was stirred with 15.5ml of a mixture of concentrated hydrochloric acid (5.5ml) and methanol (60ml), for 2.5 days at ambient temperature to yield 3-(methylamino)-1-phenyl-2-pyrazoline hydrochloride, 2.69, m.p. 174.5-176.5$^{0}$.

Example 26 : Preparation of 3-(2-Butenylamino)-1-phenyl-2-pyrazoline

A. Preparation of N-(2-butenyl)-N-(1-phenyl-2-pyrazolin-3-yl)formamide

By a procedure analogous to that of example 2A, N-(1-phenyl-2-pyrazolin-3-yl)formamide was treated with 2-butenyl bromide to yield, after recrystallisation from light petroleum (60-80$^{0}$)-ethyl acetate, N-(2-butenyl)-N-(1-phenyl- 2-pyrazolin-3-yl)formamide 3.1 g, m.p. 100$^{0}$.

B. Preparation of 3-(2-butenylamino)-1-phenyl-2-pyrazoline

By a procedure analogous to that of example 2B(b), N-(2-butenyl)-N-(1-phenyl-2-pyrazolin-3-yl)formamide (1.4 g) was stirred with 7ml of 1.0N sodium hydroxide solution and ethanol (100ml) for 1 hour at 30$^{0}$, to afford 3-(2-butenylamino)-1-phenyl-2-pyrazoline, the hydrochloride salt of which (0.71 g) had m.p. 117.2$^{0}$.

Examples 27 and 28

The following compounds were also prepared in a manner analogous to that described in Example 2B(b):-

27) 3-ethoxycarbonylethylamino-1-(3-trifluoromethylphenyl)-2-pyrazoline, m.p. 133-135$^{0}$C.

28) 3-methylamino-1-(4-methylthiophenyl)-2-pyrazoline hydrochloride, m.p. 183-185$^{0}$C.

Example 29: Preparation of N-methyl-N-[1-(3-trifluoromethylphenyl)-2-pyrazolin-3-yl] acetamide

A mixture of 3-methylamino-1-(3-trifluoromethylphenyl)-2-pyrazoline (prepared from 7 g of the hydrochloride salt) and acetic anhydride (6 ml) was heated under reflux in chloroform (50 ml) for 1.5 hours. Removal of excess solvent in vacuo, followed by addition of water precipitated the crude product. Recrystallisation from 2-propanol-water, and then light petroleum (60-80$^{o}$), afforded N-methyl-N-[1-(3-trifluoromethylphenyl)-2-pyrazolin-3-yl]acetamide, 5 g, m.p. 109-110$^{o}$.

Example 30: Preparation of N-methyl-N-[1-(3-trifluoromethylphenyl)-2-pyrazolin-3-yl]acetamide

A. Preparation of N-[1-(3-trifluoromethylphenyl)-2-pyrazolin-3-yl]acetamide

3-Amino-1-(3-trifluoromethylphenyl)-2-pyrazoline (10 g) was added in four portions to acetic anhydride (50 ml) and the mixture was stirred at ambient temperature for 1 hour to form a precipitate. Isolation of the crude precipitate followed by recrystallisation from 2-methoxyethanol afforded N-[1-(3-trifluoro-methylphenyl)-2-pyrazolin-3-yl]acetamide, 4.9 g, m.p. 248-251$^{o}$.

B. Preparation of N-methyl-N-[1-(3-trifluoromethylphenyl)-2-pyrazolin-3-yl] acetamide

Sodium hydride (50% dispersion in oil, 0.48 g) was added in portions to N-[1-(3-trifluoromethylphenyl)-2-pyrazolin-3-yl]acetamide (2.71 g) in N,N-dimethylformamide (30 ml) and the mixture stirred until the evolution of hydrogen had ceased. Iodomethane (0.75 ml) was added, and the mixture was stirred for 2 hours and then left to stand overnight. Dilution of the reaction mixture with ice-water to precipitate the crude product, followed by recrystallisation from light petroleum (60-80$^{o}$) afforded N-methyl-N-[1-(3-trifluoromethylphenyl)- 2-pyrazolin-3-yl]acetamide, 1.6 g, m.p. 108-110$^{o}$.

Example 31: Preparation of N-(2-propynyl)-N-[1-(3-trifluoromethylphenyl)-2-pyrazolin- 3-yl]acetamide

A procedure analogous to that described in example 30B, was used to prepare N-(2-propynyl)-N-[1-(3-trifluoromethylphenyl)-2-pyrazolin-3-yl]acetamide, m.p. 87$^{o}$, after recrystallisation from light petroleum (60-80$^{o}$).

Example 32: Preparation of N-Methyl-N-[1-(3-trifluoromethylphenyl)-2-pyrazolin-3-yl] propionamide

A mixture of 3-methylamino-1-(3-trifluoromethylphenyl)-2-pyrazoline (500 mg), propionic anhydride (1 ml), and chloroform (3 ml) was heated for 5 hours at 70$^{0}$, and then evaporated in vacuo. The residue was triturated with SVM, and the solvent evaporated. Fractional crystallisation of the residue from light petroleum (60-80$^{0}$) afforded N-methyl-N-[1-(3-trifluoromethylphenyl)-2-pyrazolin-3-yl]propionamide 1.2 g, m.p. 74$^{0}$.

Example 33: Preparation of N-ethoxycarbonylmethyl-N-[1-(3-trifluoromethylphenyl)-2-pyrazolin-3-yl]formamide

By a procedure analogous to that of example 1B, N-[1-(3-trifluoro methylphenyl)- 2-pyrazolin-3-yl]formamide was treated with ethyl-2-bromoacetate to yield, after recrystallisation from ethanol, N-ethoxycarbonylmethyl-N-[1-(3-trifluoromethylphenyl]-2-pyrazolin-3-yl]formamide,m.p. 123-125$^{0}$.

Example 34: Preparation of N-(4-chloro-2-butenyl)-N-[1-(3-trifluoromethylphenyl)-2-pyrazolin-3-yl]formamide

By a procedure analogous to that of example 1B, N-[1-(3-trifluoromethylphenyl)- 2-pyrazolin-3-yl]formamide was treated with 1,4-dichloro-2-butene to afford, after recrystallisation from light petroleum (60-80$^{0}$), N-(4-chloro-2-butenyl)-N-[1-(3-trifluoromethylphenyl)-2-pyrazolin-3-yl]formamide m.p. 96-98$^{0}$.

Example 35: Preparation of N-(2-propenyl)-N-[1-(3,5-ditrifluoromethyl)-2-pyrazolin-3-yl]formamide

The mono-N-substituted formamide used as a starting material in Examples 13A and 14A was also used to prepare the title compound in a manner analogous to that described in Example 1B, m.p. 106-107$^{0}$C.

Examples 36 and 37:

The following compounds were also made in a manner analogous to that described in Example 1A and 1B:-

36) N-methyl-N-[1-(4-chlorophenyl)-2-pyrazolin-3-yl]benzamide,m.p.165.1°C.

37) N-(2-ethoxycarbonylethyl)-N-[1-(3-trifluoromethylphenyl)-2-pyrazolin-3-yl]-formamide,m.p.90-92°C.

Example 38: Preparation of N-[1-(3-methylthiophenyl)-2-pyrazolin-3-yl]-N-methylacetamide

A. Preparation of N-[1-(3-methylthiophenyl)-2-pyrazolin-3-yl]acetamide

A mixture of 3-amino-1-(3-methylthiophenyl)-2-pyrazoline (1g) and acetic anhydride (5 ml) was stirred for 1 hr at room temperature to precipitate N-[1-(3-methylthiophenyl)-2-pyrazolin-3-yl]acetamide (0.4g), m.p. 183-185°C after recrystallisation from ethanol.

B. Preparation of N-[1-(3-methylthiophenyl)-2-pyrazolin-3-yl]-N-methylacetamide

By the procedure of Example 16A but using methyl iodide in place of the allyl bromide, there was obtained N -[1-(3-methylthiophenyl)-2-pyrazolin-3-yl]-N-methylacetamide, m.p. 104-106°C after recrystallisation from ethanol.

Examples 39 and 40

The following compounds (A) were prepared in a manner analogous to that described in Examples 30A, and these in turn, were converted to the compounds (B) of formula (III) in a manner analogous to that described in Example 30B.

39) A. N-[1-(3-bromophenyl)-2-pyrazolin-3-yl]acetamide, m.p. 222.5-223.5°C.
B. N-methyl-N-[1-(3-bromophenyl)-2-pyrazolin-3-yl]acetamide, m.p. 79.5-80°C.

40) A. N-[1-(3,5-ditrifluoromethylphenyl)-2-pyrazolin-3-yl]acetamide,m.p.272-273°C.
B. N-methyl-N-[1-(3-bromophenyl)-2-pyrazolin-3-yl]acetamide,m.p.123-124.5°C.

Example 41-47

The following compounds were also prepared in a manner analogous to that described in Example 30B:-

41) N-methyl-N-[1-(3-quinolyl)-2-pyrazolin-3-yl]acetamide,m.p.163.1.$^{o}$C.

42) N-propyl-N-[1-(3-trifluoromethylphenyl)-2-pyrazolin-3-yl]acetamide,m.p.35-36$^{o}$C.

43) N-ethyl-N-[1-(3-trifluoromethylphenyl)-2-pyrazolin-3-yl]acetamide,an oil.

44) N-methyl-N-[1-(3-nitrophenyl)-2-pyrazolin-3-yl]acetamide,m.p.102-104$^{o}$C.

45) N-methyl-N-[1-(4-bromo-3-trifluoromethylphenyl)-2-pyrazolin-3-yl]acetamide, m.p. 122-123$^{o}$C.

46) N-methyl-N-[1-(4-methylthiophenyl)-2-pyrazolin-3-yl]acetamide,m.p. 98.5-100.5$^{o}$C.

47) N-(2-propenyl)-N-[1-(3-methylthiophenyl)-2-pyrazolin-3-yl]acetamide,m.p. 51-52.5$^{o}$C.

Example 48

The following compound (A) was prepared in a manner analogous to that described in Example 30A, and this in turn was converted to the compound (B) of formula (III) in a manner analogous to that described in Example 30B:-

A. N-[1-(3-trifluoromethylphenyl)-2-pyrazolin-3-yl]trifluoroacetamide,m.p. 154.5-156$^{o}$C.

B. N-methyl-N-[1-(3-trifluoromethylphenyl)-2-pyrazolin-3-yl]trifluoroacetamide,m.p. 64-68$^{o}$C.

EXAMPLE A : Tablet

In one tablet

| | |
|---|---|
| Active Ingredient | 5.0 mg |
| Lactose | 82.0 mg |
| Starch | 10.0 mg |
| Povidone | 2.0 mg |
| Magnesium Stearate | 1.0 mg |

Mix together the active ingredient, lactose and starch. Granulate the powders using a solution of povidone in purified water. Dry the granules, add the magnesium stearate and compress to produce tablets, 100 mg per tablet.

RMW/KMS/DC14/16.01.84

EXAMPLE B : Ointment

| | |
|---|---|
| Active Ingredient | 1.0 g |
| White Soft Paraffin | to 100.0 g |

Disperse the active ingredient in a small volume of the vehicle. Gradually incorporate this into the bulk to produce a smooth, homogeneous product. Fill into collapsible metal tubes.

EXAMPLE C : Cream for Topical Use

| | |
|---|---|
| Active Ingredient | 1.0 g |
| Polawax GP 200 | 20.0 g |
| Lanolin Anhydrous | 2.0 g |
| White Beeswax | 2.5 g |
| Methyl Hydroxybenzoate | 0.1 g |
| Distilled Water | to 100.0 g |

Heat the Polawax, beeswax and lanolin together at $60^{0}C$. Add a solution of methyl hydroxybenzoate. Homogenise using high speed stirring. Allow the temperature to fall to $50^{0}$. Add and disperse the active ingredient. Allow to cool with slow speed stirring.

EXAMPLE D : Lotion for Topical Use

| | |
|---|---|
| Active Ingredient | 1.0 g |
| Sorbitan Monolaurate | 0.6 g |
| Polysorbate 20 | 0.6 g |
| Cetostearyl Alcohol | 1.2 g |
| Glycerin | 6.0 g |
| Methyl Hydroxybenzoate | 0.2 g |
| Purified Water B.P. | to 100.00 ml |

The methyl hydroxybenzoate and glycerin were dissolved in 70 ml of the water at $75^{0}C$. The sorbitan monolaurate, Polysorbate 20 and cetostearyl alcohol were melted together at $75^{0}C$ and added to the aqueous solution. The resulting emulsion was homogenised, allowed to cool with continuous stirring and the active ingredient added as a suspension in the remaining water. The whole was stirred until homogeneous.

EXAMPLE E : Eye Drops

| | |
|---|---|
| Active Ingredient | 0.5 g |
| Methyl Hydroxybenzoate | 0.01 g |
| Propyl Hydroxybenzoate | 0.04 g |
| Purified Water B.P. | to 100.00 ml |

The methyl and propyl hydroxybenzoates were dissolved in 70 ml purified water at 75$^{o}$ and the resulting solution then allowed to cool. The active ingredient was added next and the solution made up to 100 ml with purified water. The solution was sterilised by filtration through a membrane filter 0.22 $\mu$ pore size and packed aseptically into suitable sterile containers.

EXAMPLE F : Injection Solution

| | |
|---|---|
| Active Ingredient | 10.0 mg |
| Water for Injections B.P. | to 1.0 ml |

The active ingredient was dissolved in half of the Water for Injection and then made up to volume and sterilised by filtration. The resulting solution was distributed into ampoules under a aseptic conditions.

STABILITY

Freshly prepared samples of the compound of Example 29/30B and the compound 2B (the latter also being disclosed in European Specification 55 418) were stored separately at normal room temperature in sealed containers and analysed at regular intervals by T.l.c. The compound of Example 2B showed significant decomposition after six months had elapsed, whereas the compound of Example 29/30B had showed no significant decomposition, 2 years from the outset of the Experiment.

EXAMPLE I : Inhibition of Lipoxygenase and Cyclooxygenase

In an enzyme assay according to a method closely based on that of P. Borgeat et al (J. Biol. Chem. 251 (1976) 7816-20), compounds of the invention were found to have an $IC_{50}$ (uM) for inhibition of each of lipoxygenase and cyclo-oxygenase as indicated in Table I.

TABLE I

| Compound of | $IC_{50}$ (uM) | |
|---|---|---|
| Example No. | Cyclooxygenase | Lipoxygenase |
| 3A | 5 | 2 |
| 4A | 3 | 0.5 |
| 5A | 10 | 1 |
| 8A | 8 | 2 |
| 9A | 2 | 1.5 |
| 18B | 2 | 5 |
| 29&30B | 1 | 1 |
| 32 | >10 | 1 |
| 34 | 10 | 3 |
| 45 | 1 | 1 |

Example II : In vivo Activity

The compound of Example 29/30B was subjected to the following tests. Results are given in Table II as $ED_{50}$ mg/kg upon oral administration.

1) Prostaglandin synthesis and leucocyte accumulation

Inflammatory exudates were collected 24 hours after the subcutaneous implantation in male rats (180-220g) of sterile polyester sponges impregnated with 2% carrageenin. Prostaglandin $E_2$ concentrations in sponge exudates were measured by bioassay and total leucocyte numbers were estimated using counting chambers and phase contrast microscopy. Drugs were administered orally in aqueous solution to groups of five animals at the time of sponge implantation, 5-8 hours later and 3 hours before sponge removal. Results are expressed as a percentage of control values obtained from similar groups of animals receiving vehicle alone. $ED_{50}$ values were calculated for active compounds. The full dose-response data for lead compounds were subjected to regression analysis and $ED_{50}$ values with 95% confidence limits were obtained.

2) Oedema

Oedema was induced by the sub-plantar injection of 2% carrageenin (0.1 ml) in the hind paws of female rats (100-130g). Swelling was measured 1-4 hours following carrageenin injection using a micrometer gauge. Drugs in aqueous solution were administered orally to groups of five rats at the time of carrageenin injection. Mean swelling was expressed as a percentage of control values obtained from similar groups of animals receiving vehicle alone.

3) Pain

Writhing was induced in groups of 5 female mice (20-25g) following the intraperitoneal injection of phenylbenzoquinone (PBQ; 2.5 mg/kg). Anti-nociceptive effects of drugs were assessed by observing the reduction in writhing responses 10-12½ min after PBQ injection. Drugs were administered orally in aqueous solution 1 hour before PBQ injection and drug effects were expressed as a percentage of control values obtained from animals receiving vehicle alone.

Table II

| Test | ED$_{50}$ (mg/kg p.o.)<br>Compound of Example 29/30B |
|---|---|
| Prostaglandin Production (rat) | 35 |
| Leucocyte Accumulation (rat) | 57 |
| Carrageenin Oedema (rat) | 15 |
| PBQ Writhing (mouse) | 45 |

Claims:-

1) A process for the preparation of a compound of formula (I)

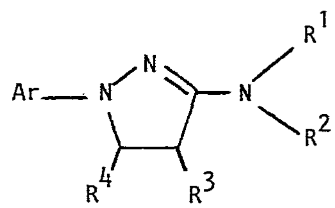

(I)

wherein,

Ar is an aryl group, optionally substituted by one or more suitable substituents;

$R^1$ is alkyl, alkenyl, alkynyl, alkoxy, aryl, aralkyl, aralkenyl or aralkynyl;

$R^2$ is hydrogen, alkyl or aralkyl; and

$R^3$ and $R^4$ are the same or different, each being hydrogen or alkyl; or an acid addition salt thereof,

which comprises

(i) reacting of a compound of formula (II) or an acid addition salt thereof

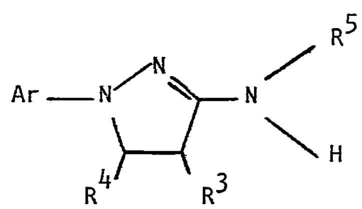

(II)

wherein,

Ar is an aryl group, optionally substituted by one or more suitable substituents;

$R^3$ and $R^4$ are the same or different, and each may be alkyl or hydrogen; and

$R^5$ is acyl or aroyl;

with a compound serving to effect substitution of a group $R^1$ as defined in formula (I) on the amino group attached to the 3-position of the pyrazoline ring in the compound of formula (II), thereby to form a compound of formula (III) or an acid addition salt thereof

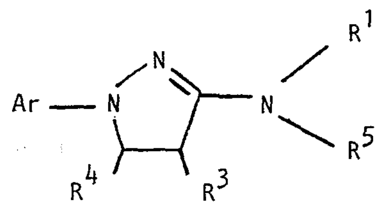

(III)

wherein Ar, $R^1$, $R^3$, $R^4$ and $R^5$ are as hereinbefore defined;

and converting the compound of formula (III) to a compound of formula (I); or an acid addition salt thereof.

2) A process as claimed in claim 1, wherein the intermediate compound of formula (III) or an acid addition salt thereof is produced in situ.

3) A process as claimed in claim 1, wherein the intermediate compound of formula (III) or an acid addition salt thereof is isolated prior to its conversion of a compound of formula (I) or an acid addition salt thereof.

4) A process according to any preceding claim when used for the preparation of a compound of formula (I) wherein

Ar represents an unsubstituted pyridyl, quinolyl or naphthyl group or a phenyl group substituted by one or more groups selected from $C_{1-4}$ alkyl, trihalo-(eg. trifluoro-) $C_{1-4}$ alkyl, halo, nitro and $C_{1-4}$ alkylthio;

$R^1$ represents a group selected from $C_{1-4}$ alkyl, $C_{3-6}$ alkenyl, $C_{3-6}$ alkynyl, phenyl-$C_{1-4}$ alkyl (eg. benzyl) (the phenylalkyl group being optionally substituted by one or more groups selected from halo, $C_{1-4}$ alkoxy and hydroxy), $C_{1-4}$ alkoxycarbonyl-$C_{1-4}$ alkyl, cyclo-$C_{3-6}$ alkyl, N,N-di-$C_{1-4}$-alkylamino-$C_{1-4}$ alkyl and N,N-di- $C_{1-4}$ alkylcarbamoyl-$C_{1-4}$ alkyl;

$R^2$ represents hydrogen; and

$R^3$ and $R^4$ both represent hydrogen;

and acid addition salts thereof.

5) A process according to any preceding claim when used to prepare 3-methylamino-1-(3-trifluoromethylphenyl)-2-pyrazoline or an acid addition salt thereof.

6) A compound of formula (IIIA)

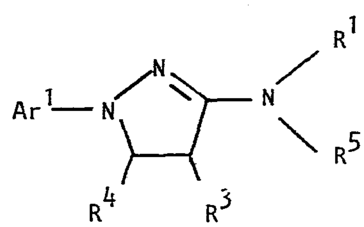

(IIIA)

wherein,

$Ar^1$ is an aryl group optionally substituted by one or more suitable substituents provided that when $Ar^1$ represents phenyl then the phenyl group bears at least one suitable substituent;

$R^1$ is alkyl, alkenyl, alkynyl, aryl, aralkyl, aralkenyl or aralkynyl;

$R^5$ is acyl or aroyl; and

$R^3$ and $R^4$ are the same or different, each being hydrogen or alkyl; and pharmacologically acceptable acid addition salts thereof.

7) A compound as claimed in claim 6 wherein

$Ar^1$ represents an unsubstituted pyridyl, quinolyl or naphthyl group or a phenyl group each of which is substituted by one or more groups selected from $C_{1-4}$ alkyl, trihalo-(eg. trifluoro-) $C_{1-4}$ alkyl, halo, nitro and $C_{1-4}$ alkylthio;

$R^1$ represents a group selected from $C_{1-4}$ alkyl, $C_{3-6}$ alkenyl, $C_{3-6}$ alkynyl, phenyl $C_{1-4}$ alkyl (eg. benzyl) (the phenylalkyl group being optionally substituted by one or more groups selected from halo, $C_{1-4}$ alkoxy and $C_{1-4}$ alkanoyloxy), $C_{1-4}$ alkoxycarbonyl-$C_{1-4}$ alkyl, cyclo-$C_{3-6}$ alkyl, N,N-di-$C_{1-4}$-alkylamino-$C_{1-4}$ alkyl and N,N-di-$C_{1-4}$ alkylcarbamoyl-$C_{1-4}$ alkyl;

$R^5$ represents a group of formula -COQ wherein Q represents hydrogen or $C_{1-4}$ alkyl (optionally substituted by one or more halogen atoms) or phenyl; and

$R^3$ and $R^4$ both represent hydrogen;

and pharmacologically acceptable acid addition salts thereof.

8) A compound as claimed in claim 6 or claim 7 wherein $Ar^1$ is a phenyl group substituted in the 3- position, and pharmacologically acceptable acid addition salts thereof.

9) N-methyl-N-[1-(3-trifluoromethylphenyl)-2-pyrazolin-3-yl]acetamide and pharmacologically acceptable acid addition salts thereof.

10) A pharmaceutical formulation comprising a compound as claimed in any of claims 6-9 or a pharmacologically acceptable acid addition salt thereof, and a pharmaceutically acceptable carrier therefor.

11) A compound of formula (IIIA) as claimed in claim 6 or a pharmacologically acceptable acid addition salt thereof for use in a method of inhibiting the lipoxygenase and cyclooxygenase pathways of the arachadonic acid metabolism in a mamml such as man.

12) A compound of formula (IIIA) as claimed in claim 6, or a pharmacologically acceptable acid addition salt thereof for use in a method of prophylaxis or treatment, by therapy, of a mammal such as man.

13) A compound of formula (IIIA) as claimed in claim 6, or a pharmacologically acceptable acid addition salt thereof for use in relief of rheumatoid arthritis, rheumatoid spondylitis, osteoarthritis, gouty arthritis and other arthritic conditions, inflammed joints, psoriasis, eczema, other inflammatory skin conditions, inflammatory eye conditions including conjunctivitis, pyresis and other conditions associated with inflamation and pain, or for the reduction of tissue necrosis in chronic inflammation, the suppression of tissue rejection following transplant surgery or the prophylaxis or treatment of ulcerative colitis.

14) A compound of formula (IIIA) as claimed in claim 9, or a pharmacologically acceptable acid addition salt thereof, for use in the treatment or prophylaxis of allergic conditions and airway inflammatory conditions such as asthma and of asthma having a non-allergic origin and bronchitis.

0119450

European Patent Office

# EUROPEAN SEARCH REPORT

Application number

EP 84 10 1395

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP-A-0 022 578 (THE WELLCOME FOUNDATION LTD.)<br>* Claims * | 1-14 | C 07 D 231/06<br>C 07 D 401/04<br>A 61 K 31/415 |
| D,X | EP-A-0 055 418 (THE WELLCOME FOUNDATION LTD.)<br>* Pages 8,9; claims * | 1-14 | |
| D,A | SOCIETE CHIMIQUE DE FRANCE, BULLETIN, 1970, pages 1571-1576<br>* Pages 1574-1575 * | 1-6 | |
| | | | TECHNICAL FIELDS SEARCHED (Int. Cl. 3) |
| | | | C 07 D 231/00<br>C 07 D 401/00 |

The present search report has been drawn up for all claims

| Place of search | Date of completion of the search | Examiner |
|---|---|---|
| THE HAGUE | 08-06-1984 | MAISONNEUVE J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82